# EUROPEAN PATENT APPLICATION

(11) **EP 4 155 709 A1**
(43) Date of publication of application: **29.03.2023**
(21) Application number: 21199383.7
(22) Date of filing: 28.09.2021
(51) Int. Cl.: G01N 1/14, G01N 1/18, B01L 3/00, C12M 1/32, G01N 1/00, G01N 21/77, G01N 35/00, G01N 1/10

(54) **ON-LINE BIOMOLECULAR INTERACTION ANALYSIS FOR MONITORING OR CONTROLLING BIOPROCESSES**

(71) Applicant: Bayer AG, 51373 Leverkusen (DE)
(72) Inventor: The designation of the inventor has not yet been filed
(74) Representative: BIP Patents

(57) **Abstract**

The invention relates to a system for measuring one or more analytes in discrete fluid samples from a vessel adapted to contain a fluid comprising one or more sensors (5) for the measurement of the analytes (53) in a contact volume of the fluid sample, an analysis module (55) comprising at least one main sampling line in fluidic connection with the vessel and one or more pumps in fluidic connection with said main sampling line(s) and adapted to selectively pump fluid from in the main sampling line away from the vessel, at least one fluid distribution tubing (30) in fluidic connection with the analysis module, a manifold (20) comprising a body (25), and in the body (25), for each sensor (5), one fluidic cell comprising one fluid distribution channel (21) and a collecting channel (22a, 22b) in fluidic communication with the fluid distribution channel (21), wherein the fluid distribution tubing (30) is fitted in the fluid distribution channel (21) so that it is emerging in the collecting channel (22) and so that the sensor (5) can be contacted with at least a drop (10) of the fluid sample emerging at the top of the fluid distribution tubing (30) as the contact volume or wherein the fluid distribution tubing (30) is fitted in the fluid distribution channel (21) in fluidic connection with a measurement well (40), so the measurement well (40) can be filled with the fluid sample to create the contact volume and the sensor (5) can be dipped in the contact volume for measurement, and wherein said collecting channels (22a, 22b) are plunging so the fluid sample is collected per gravity.

## Description

### OBJECT OF THE INVENTION

The invention relates to a solution for monitoring or controlling bioprocesses by way of withdrawing discrete fluid samples from a reactor and performing on-line analysis of the said sample using a sensor based on in particular on label-free biomolecular interaction analysis using a fluidic manifold.

### BACKGROUND OF THE INVENTION

In-line and on-line monitoring of bioprocesses is yet limited to spectroscopic or electrochemical sensors. This is a limitation regarding the ability to quantify and monitor specific biological compounds. Current biopharmaceutical processes in development (e.g. monoclonal antibodies, recombinant proteins, vaccines, cell and gene therapies) involve a large panel of product quality attributes and process parameters that are biologically active proteins (e.g. proteins produced by the cells or present in cell culture media) which cannot be monitored on-line by existing technologies. Biosensors based on label-free protein interaction analysis or more generally bioanalytical tools may provide further valuable information for process monitoring and control.

Several off-line label-free protein biosensors have been commercialized to date based on different principles : Surface Plasmon resonance (Biacore - Cytiva), Bio-Layer Interferometry (Octet - Sartorius, Gator+ - Gator bio), Quartz Cristal Microbalance (Q-Sense - Biolin Scientific), Grating-Coupled Interferometry (Wave - Creoptix). In comparison to classical ELISA bioassays, involving a lot of manual steps and long (hours) time-to-results, those systems can perform faster protein quantitative assays and evaluate kinetic parameters of a biological interaction between an analyte of interest binding to a ligand pre-functionalized onto the biosensor. Consequently, they can be used to evaluate the biological activity of protein compounds toward a dedicated ligand.

However, those label-free biosensors are currently only used for off- or at-line analysis of bioprocesses samples (sample analysis after manual sampling, transport and preparation, potentially partly automated with liquid handlers). They are therefore not directly connected to the process ("on-line"), and thus cannot be used for automated process monitoring and control. There is a need for rapid (time-to-result <30 mins) and automated protein analysis with low sample and low reagents volume consumptions and without the help of a secondary liquid handling machine/pipetting robot.

The use of such bioanalytical tools for on-line monitoring or controlling of bioprocesses could speed up process development and increase in-process control capabilities. For example, the data obtained from the sensor (e.g. protein titer, kinetic parameters, protein activity) at defined timepoints in a process can be used as input in uni- or multivariate model(s) used to control the process.

### SUMMARY OF THE INVENTION

The object underlying the invention is achieved by the combination of features according to independent Claims 1, 10 and 14. Exemplary embodiments of the invention can be gathered from the respective dependent claims.

The invention is described in more detail without any division within the subject matter of the invention (process and system). The explanations below are intended to be applicable analogously to all of the subject matters of the invention, in either context (process or system).

The solution of the invention relies on the use of a fluidic system comprising a fluidic manifold for contacting one or more sensors with the sample fluid derived from a process.

The process may be an industrial process and/or a bioprocess, such as a biotechnological process. The process may involve chemical or microbiological conversion of material in conjunction with the transfer of heat, mass, and energy. The process may be a batch process, e.g., a fed-batch bioprocess or perfusion process. The process may involve producing cells for use in, or to host, the product. More particularly, the cells may host the product, or the cells may be (part of) the product.

The terms "sample fluid" or "fluid sample" as used therein refer to a solution containing analytes, e. g. protein(s), to be measured by the sensor(s). Additionally, the sample fluid may include the fluid used in the process for producing the desired product. The sample fluid may include starting material for the process. More particularly, the sample fluid may include a medium and/or biological material (e.g., a cell culture medium).

The term "vessel" as used therein refers to a fermenter, a bioreactor, a sampling cup, a sampling loop or any other vessel containing the sample fluid to be analyzed.

The solution of the invention is therefore a system for measuring analytes in discrete fluid samples from a vessel adapted to contain a fluid comprising:
- one or more sensors (5) for the measurement of the analytes (53) in a contact volume of the fluid sample;
- an analysis module (55) comprising at least a main sampling line in fluidic connection with the vessel, one or more pumps in fluidic connection with said main sampling line(s) and adapted to selectively pump fluid from in the main sampling line away from the vessel;
- at least one fluid distribution tubing (30) in fluidic connection with the analysis module (55);
- a manifold (20) comprising a body (25), and in the body (25), for each sensor (5), one fluidic cell comprising one fluid distribution channel (21) and a collecting channel (22a, 22b) in fluidic communication with the fluid distribution channel (21);
- wherein the fluid distribution tubing (30) is fitted in the fluid distribution channel (21) so that it is emerging in the collecting channel (22) and so that the sensor (5) can be contacted with at least a drop (10) of the fluid sample emerging at the top of the fluid distribution tubing (30);
- or
   wherein the fluid distribution tubing (30) is fitted in the fluid distribution channel (21) in fluidic connection with a measurement well (40), so the measurement well (40) is filled with the fluid sample to create the contact volume and the sensor (5) can be dipped in the fluid sample for measurement;
- and wherein said collecting channels (22a, 22b) are plunging so the fluid sample can be collected per gravity.

In an embodiment, the body (25) further comprises a main waste channel (26) or a main waste funnel (27) in fluidic connection with the collecting channels (22).

In an embodiment the sensor is and optical sensor, preferred a biosensor. A biosensor is an analytical sensor comprising a biological reagent. Biosensors combine a detector (i.e. biorecognition elements interacting with the measured analyte) and a transducer which converts biological interactions into a measurable signal.

In a particular embodiment the sensor is an optical biosensor. The biosensor probe consisting in an optical fiber (51) based sensor as described in US 2007/0070356A1. Also, other biosensors allowing label-free bioanalyses, may be used. For example, biosensors based on surface plasmon resonance (e.g. as described in US2004/0186359), localized surface plasmon resonance (e.g. as described in WO 2017/046179A1) or focal molography (e.g. as described in EP2805149A1) could be envisioned for the application.

In an embodiment a plurality of sensors is used.

In an embodiment, an array of sensors is used. The one or more sensors are typically mounted on a support and 2 or 3 axis positioning system (also referred to as a sensor transport module) addressing the sensors to dedicated position in the manifold, sensor plate (in order to load new sensors), microtiter plate (containing reagents) or any other desired position.

For example, off-line systems for biomolecular interaction analysis using the technique of BIO-LAYER INTERFEROMETRY (BLI), wherein analyses are performed by dipping 8 aligned sensors arranged on a movable solid support and transport system in a row of 8 wells arranged in a 96-wells plate, can be used. Commercially available BLI systems include: "Octet RED96/Octet R" systems (Sartorius) and "Gator Prime/Gator plus" systems (Gator bio).

The system of the invention comprises a fluidic system dedicated for the automated delivery of the fluid sample from the process to one or more biosensors, contacting the sensors with the fluid, followed by signal detection with or without a preceding signal amplification step.

The term "fluidic system" as used therein refers to a device capable of transporting the fluid from a vessel to and from the sensor(s). The fluidic system comprises the pumps, the valves, the fluid distribution channels, the fluid distribution tubing, the optional measurement wells, the collecting channels and the waste channel or funnel.

In an embodiment, the fluid sample may be diluted with a defined volume of buffer or mixed with a reagent before filling into the manifold via the sampling line.

In an embodiment, the main sampling line and / or the fluid distribution tubing are made of PTFE, PEEK, PFA, FEP or silicon-based tubing, typically commercially available tubing with an inner diameter ranging from 0.1 to 20 mm, preferentially 0.25 to 1 mm.

The analysis module is adapted to selectively pump fluid away from the vessel into the manifold. Commercial analysis modules may be used. In an embodiment, the system may comprise one or more filters filtering the sample before it enters the manifold. Typically, such filters are placed at vent ports.

In an embodiment, high-throughput automated bioreactor and micro-bioreactor system (e.g. Sartorius AmbR 15 and AmbR 250, mp2 labs biolector XP) (60) including a liquid handling system (62) and a sample cup (64) and allowing to run multiple vessels (61) in parallel and automatically perform regular sampling and feed addition with a sampling device (63) can be used. The sampling device (63) may implement an automated pipetting system and/or carry pipet tips. Commercial analysis module (e.g. Sartorius analysis module) (65) can be used to automatically deliver the sample fluid from the sample cup (64) to the manifold (20) by fluidic connection with at least one fluid distribution tubing (30). Other bioprocess autosampling systems may be used as analysis module for automated sample collection and distribution of the sample to the manifold (20). Commercial bioprocess autosamplers comprise, but are not limited to, the following systems: Numera (Securecell), MAST (Lonza), SegFlow (Flownamics), BioPAT (Sartorius). Analysis module may require gas filters for maintenance of sterility. In an embodiment, each filter comprises a sterilizing grade gas filter with a 0.2 or 0.45 µm membrane.

One fluid distribution channel (21) in fluidic connection with its collecting channel (22) is together referred to as a fluidic cell of the manifold.

In an embodiment, a plurality of sensors is used. It is preferred that the sensors are arranged in an array, for example in a row.

In an embodiment, the manifold comprises an arrangement of a plurality of fluidic cells. For reason of compatibility with the commercial "Octet RED96/Octet R8" preferably 8 identical fluidic cells in alignment were chosen in an example.

The person skilled in the art will appreciate that the number of fluidic cells is discretionary. He will also appreciate, that the geometrical arrangement of the fluidic cells in the manifold are governed by practicability, although a manifold comprising one array of fluidic cells is preferred.

In an embodiment, the number of fluidic cells is the same as the number of sensors.

In an embodiment the geometrical arrangement of the fluidic cells is the same as the arrangement of the sensors.

In an embodiment one or more arrays of fluidic cells and / or sensors are used.

In an embodiment, the one or more fluid distribution channel (21) is in fluidic connection with a main distribution channel (32), which is in fluidic connection with the main sampling line.

In this embodiment, it is preferred that the main distribution channel (32) is dimensioned so that the main sampling line can be fitted and maintained in the main distribution channel (32). In an example, the main distribution channel (32) is horizontal or nearly horizontal and the one or more fluid distribution channels (21) are vertical or nearly vertical. It is preferred that the fluid distribution channels (21) are identical in dimension and aligned to each other to form an array. In an embodiment the fluid distribution channels (21) are distributed along the main distribution channel (32) at regular distance. In an embodiment, the main distribution channel (32) is dimensioned so that the flow is identical or nearly identical in all the fluid distribution channels (21).

In an alternative embodiment, at least one fluid distribution tubing (30) is in fluidic connection with a analysis module (55) comprising one or more pumps and / or valves in fluidic connection with the main sampling line connected to one or more vessels. In this embodiment, it is preferred that the one or more fluid distribution channel (21) is dimensioned so that the fluid distribution tubing (30) can be fitted and maintained in the fluid distribution channel (21). In an embodiment, the one or more fluid distribution channel (21) are vertical or nearly vertical in the body.

In an embodiment, the fluid distribution tubing (30) is mounted in the fluid distribution channel (21) and maintained by a fitting (31) as a fluidic connector. Other type of fluidic connectors can be envisioned for fixing the tubing.

In an embodiment, the pumps may be peristaltic pump, diaphragm pump, piston pump, syringe pump or a combination thereof.

In an embodiment, the system comprises an automated flow control system adapted to:
(a) configure and store a sample filling configuration for the analysis module, in particular for its one or more pumps and / or valves, the sample filling configuration comprising a prescribed filling period and / or a prescribed filling volume;
(b) automatically operate said sampling system in a sampling direction away from the vessel for the filling of the sample fluid into the manifold for the prescribed using the filling configuration;
(c) configure and store a contact configuration for the analysis module, in which sample flow is stopped for a prescribed contact period, during which an analysis is automatically launched and the sensor (5) is contacted with the contact volume;
(d) automatically operate said sampling system for a prescribed contact period using the contact configuration; and / or
(e) configure and store a cleaning configuration for the system, preferred for the analysis module, the cleaning being achieved by pushing the sample fluid and / or cleaning fluid away from the sensor(s) into the collecting channels (22), said cleaning configuration comprising one or more cleaning periods and / or one or more cleaning volumes;
(f) automatically operate the system using the cleaning configuration.

The term "cleaning fluid" as used therein refers to cleaning solution and / or a buffer solution used for cleaning the fluidic system between the analysis of samples.

The term "cleaning period" refers to the time or volume needed to flush and clean the fluidic system.

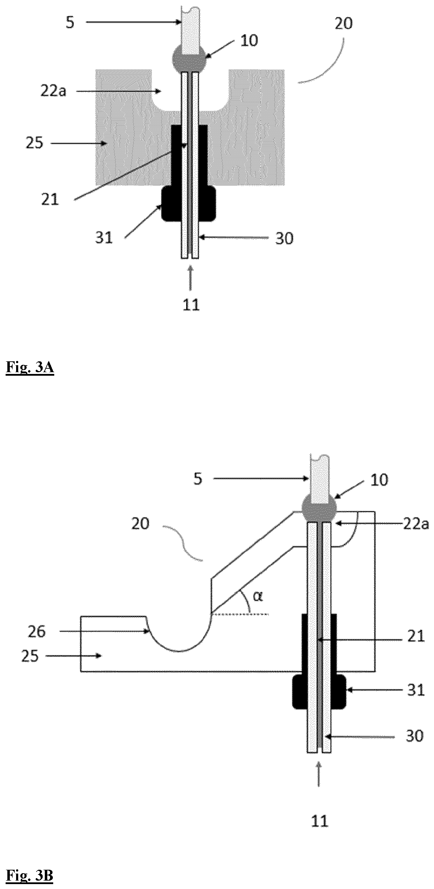

In an embodiment (Fig 3A and 3B), the fluid distribution tubing (30) is fitted in the fluid distribution channel (21) so that it is emerging in the fluid collecting well (22) and that the sensor (5) can be contacted with at least a steady drop (10) of the fluid emerging at the top of the fluid distribution tubing (30). In this embodiment the sensor (5) is positioned so that the steady drop (10) of the fluid is emerging at the top of the fluid distribution tubing (30) and contacting with the sensor (5) is achieved during analyses.

In this embodiment, the filling period is the time required to generate the steady drop at the predefined flow rate for the sample fluid. The sample fluid is typically forwarded into the fluidic system at a flow rate ranging from 0.01 to 1 mL/min, preferred 0.1 to 0,8 mL/min, most preferred 0.5 mL/min, depending on the tubing dimensions. For the generation of the steady drop the sensors may be positioned at approximatively 2 mm of the outlet of the fluid distribution tubing. The one or more pumps of the analysis module are stopped for a prescribed contact period to prevent the fluid from flowing into the collecting channel (22) for the sensor contact period. Optimal distance, flow rate and time for the generation of drop are typically established experimentally for the specific embodiment used.

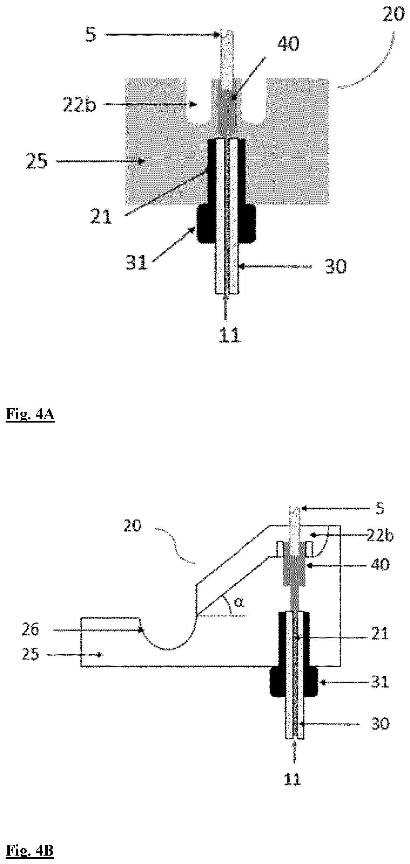

In an alternative embodiments (Fig. 4A, 4B), the fluid distribution tubing (30) is fitted in the fluid distribution channel (21) in fluidic connection with a measurement well (40), so the measuring well (40) is filled with the fluid. In this embodiment the sensor is positioned to dip into the fluid sample. In this embodiment the filling period is typically the time required to fill the measurement well (40). In this particular embodiment, the form of the measurement well (40) is preferably cylindrical with a volume from 10 to 500 µl, preferred from 100 to 400 µl, wherein the dimensions of the measurement well (40) allow the convenient positioning of the sensor or array of sensors in the fluid volume filled in the measurement well (40). Typically, the measurement well (40) shows a diameter from 1 to 5 mm, preferred 3 mm and a height from 5 to 20 mm, preferred 15 mm.

In an embodiment (Fig. 4A, 4B), the measurement well (40) is preferably overflowing into a collecting channel (22b) encompassing the measurement well (40). The collecting channel (22b) is also used for flushing with cleaning fluid.

For all embodiments described above, cleaning is conducted at the end of the contact period: the automated control system operates the one or more pumps and/ valves of the analysis module (55) to flush the fluidic system with the cleaning fluid for a predefined cleaning period, preferably in a continuous cleaning flow. The cleaning fluid flows into the collecting channel and pours out per gravity into a main waste channel or a main waste funnel in fluidic connection with the collecting channels

In an embodiment the collecting channel (24) is plunging into the main waste channel (26) or the main waste funnel (27) with an angle α of 5 to 80 degree, preferred 20 to 40 degree, most preferred 30 degree to the vertical (plumb line).

In an embodiment, the collecting channel (22a, 22b) may be a pipe, U-shaped or V-shaped. It is preferred U-shaped. The dimension of the collecting channel (22a, 22b) is discretionary. In a preferred embodiment the diameter of the collecting channel (22a, 22b) is from 3 to 10 mm.

It is clear to the man skilled in the art, that form and dimension of the collecting channel (22a, 22b) is discretionary as it is intended to collect the fluid drop after contact is ended and the cleaning fluid is flushed into the fluidic system.

In an embodiment, the main waste channel (26) is U-shaped or V-shaped, preferred U-shaped; the dimension of the main waste channel (26) is discretionary.

In case a main waste funnel (27) is used, its dimensions and form are discretionary.

In an embodiment, cleaning fluid is forwarded into the fluidic system at a flow rate ranging from 1 to 10 mL/min, preferred 1 to 5 mL/min, most preferred 5 mL/min.

It is clear to the man skilled in the art, that the fluid flowing rates are typically set for controlled filling and overflowing of the measurement well (40) if used, without splashing or overflowing of the collecting channels (22a, 22b) and / or main waste channel (26) or main waste funnel (27).

The person skilled in the art will also appreciate, that the dimensions of a fluidic cell in the manifold as well as flow rates used for the transport of fluids in the manifold are governed by practicability and the design of the specific embodiment.

In an embodiment, the body of the manifold is made of inert plastic material. Different material can be used, encompassing but not restricted to: POM (Delrin/Acetal), Nylon, PEEK, PTFE (Teflon), Acrylonitrile butadiene styrene (ABS), Acrylic/Plexiglas (PMMA), Polycarbonate (PC), Polysulphone (PSU), Polyetherimide (PEI, Ultem), Cyclic Olefin Copolymer (COC).

In an embodiment, the body of the manifold is produced by way of CNC machining or/and molding or/and diffusion bonding. Preferably, a PEEK manifold produced by CNC machining is used.

In an embodiment, the sensors are mounted on a sensor positioning system. An automated control system is adapted to position the sensors (5) for contacting with the fluid sample by controlling the sensor positioning system.

In an embodiment, the sensors are mounted on a 2 or 3-axis linear sensor positioning system and can be spatially addressed to dedicated positions in the fluidic cells (one sensor per fluidic cell).

In an embodiment, an automated control system is adapted to position the sensors for contacting with the fluid sample by controlling the sensor positioning system.

A further object of the invention is a manifold (20) adapted to provide at least one discrete volume of a fluid sample for contacting with at least one sensor (5) in a system for measuring analytes, said manifold (20) comprising:
- a body (25),
- in the body (25), for each sensor (5), one fluidic cell comprising one fluid distribution channel (21) and a collecting channel (22a, 22b) in fluidic communication with the fluid distribution channel (21);
   wherein a fluid distribution tubing (30) can be fitted in the fluid distribution channel (21) so that it is emerging in the collecting channel (22) and so that the sensor (5) can be contacted with at least a drop (10) of the fluid sample emerging at the top of the fluid distribution tubing (30);
   or
      wherein the fluid distribution tubing (30) can be fitted in the fluid distribution channel (21) in fluidic connection with a measurement well (40), so the measurement well (40) is filled with the fluid sample and the sensor (5) is dipped in the fluid sample for measurement;
      - and wherein said collecting channels (22) are plunging so the fluid sample is collected per gravity.

In an embodiment the manifold (20) comprises an arrangement of a plurality of fluidic cells, preferably in a row.

The analysis may comprise further steps requiring moving the biosensor in reaction wells using the sensor positioning system (e.g. one or more dedicated wells on a microtiter plate), said wells containing dedicated reagent(s), buffer(s) and/or regeneration solutions. In addition to addressing the fluidic cells, the sensors may spatially address dedicated well positions according to a predefined analysis protocol: for example for ligand capture or immobilization step, washing step with a buffer solution, secondary labeling step, enzymatic detection step, signal amplification step, sensor regeneration step, sensor calibration measurements or the like. In other words, the system of the invention allows signal detection with or without amplification steps.

In an embodiment, the system of the invention therefore comprises a microtiter plate comprising wells in the same arrangement as the manifold, said wells containing dedicated reagents as required by the analysis protocol. The microtiter plate may be positioned in a drawer for better handling.

In an example, the Octet RED96/Octet R (60) is used, wherein the manifold of the present invention is positioned in alignment with the well plate (61) so that both the manifold and the well plate can be addressed by the sensor transport module.

It is obvious to the person skilled in the art that the solution of the present invention may be used for analysis of multiplexing capabilities (e.g. multiple bioreactors).

The solution of the invention is flexible and enables the evaluation of multiple analytical parameters in parallel by a single or/and multiple steps detection scheme:
- first step in-process (e.g. on-line) affinity capture of the analyte (53) by a ligand (52) pre-immobilized on the sensor (5) using the fluidic system of the invention,
- followed by signal detection with or without signal amplification by moving the sensor (5) in another position (e.g. a dedicated well on a 96 wells plate) where it is brought in contact to a solution containing dedicated reagent.

The first capture step can also be directly used for on-line protein analysis of the analyte within the fluid sample.

The biosensors may address the well plate for multiple-step analysis in dedicated buffer.

A further object of the invention is a method for measuring analytes in discrete fluid samples from a vessel adapted to contain a fluid comprising the steps of:
a) pumping the sample fluid from the vessel into a manifold (20) as described above to create a contact volume of fluid sample in the fluidic cells of the manifold (20);
b) contacting the sensor (5) with the contact volume and conducting measurement for a contact period;
c) pushing the fluid sample into the collecting channels (22a, 22b) and eliminating the fluid sample per gravity;
d) outputting and / or forwarding measurement on a user interface or to an on-line process monitoring device.

The method may further comprise cleaning the system by way of pumping sample fluid and / or cleaning fluid through the fluidic cell into the collecting channels (22a, 22b).

In an embodiment, the method steps are reiterated in a timely defined manner so that in-process samples can be analyzed automatically

The method of the invention is particularly useful for measuring analytes in a biological process, in particular for on-line process monitoring.

The output of the method of the invention may be used for process control. In particular, the output of the sample analysis can be used as an input variable in a process control model.

The solution of the invention solves several issues:
a) low sample volume consumption;
b) low reagent volume consumption (the reagents are kept in the well plate and re-used for multiple analysis);
c) Automated sample processing (transport, dilution, reagent mixing) and analysis with a biosensor, without the need of external microtiter plate handling robot.

This configuration offers a very low (≤50 uL) sample volume consumption per analysis and is therefore advantageous for monitoring small-scale bioprocesses.

Additionally, the system may comprise different biosensors for parallel evaluation of different attributes related to one or several analytes. This configuration allows minimum reagent and calibrator consumption as the reagents placed in the well plate can be used for multiple analyses.

The underlying sensing principle is based on Bio-layer interferometry (BLI), allowing for label-free quantification of analytes with time of analysis ranging from seconds to minutes, potentially allowing in-process control based on analysis.

Analytical measurements from the biosensor performed at given timepoint of the process may also be used as an input in a process control model used to determine process outputs in a biopharmaceutical and/or biological process. A process output may be a key performance indicator (e.g., product titer), a product specific (critical) quality attribute, CQA, (e.g., glycosylation profile), or a critical process parameter, CPP (e.g. concentration and/or biological activity of a given protein added into the culture medium). The method may further comprise initiating a control action via a process control device in order to optimize the process output value. The control action may include increasing or decreasing supply of a nutrient. For example, WO2020173844A1 (Multivariate process chart to control a process to produce a chemical, pharmaceutical, biopharmaceutical and/or biological product) and WO2017199006A1 (automated bioprocess development) describe the use of such process control models in bioprocesses.

The use of terms "a" and "an" and "the" and similar referents in the context of describing the invention (especially in the context of the following claims) are to be construed to cover both the singular and the plural, unless otherwise indicated herein or clearly contradicted by context. The terms "comprising", "including", "having", and "containing" are to be construed as open-ended terms (i.e. meaning "including but not limited to") unless otherwise noted.

The use of any and all examples, or exemplary language (e.g., "such as") provided herein, is intended merely to better illuminate the invention and does not pose a limitation on the scope of the invention unless otherwise claimed. No language in the specifications should be constructed as indicating any non-claimed element as essential to the practice of the invention.

Preferred embodiments of this invention are described, including the best mode known to the inventors for carrying out the invention. Variations of those preferred embodiments can become apparent to those of ordinary skilled artisans to employ such variations as appropriate, and the inventors intend for the inventions to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

### Examples

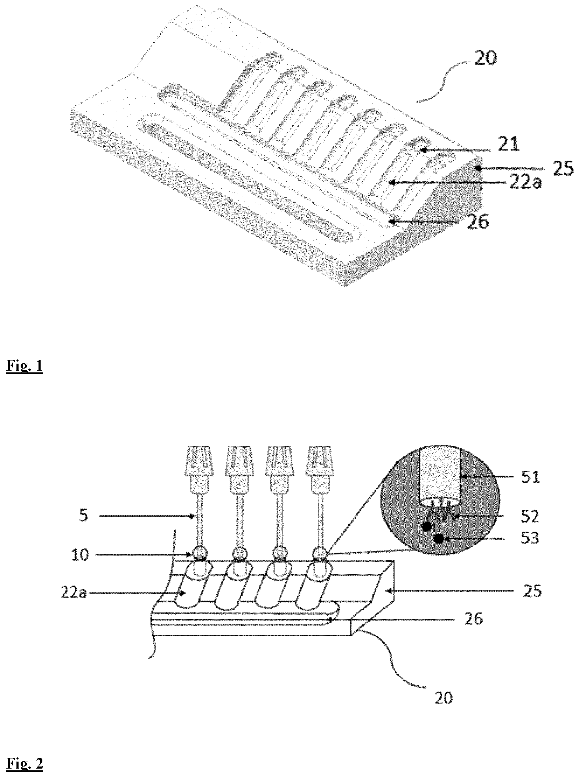

Fig. 1 shows a 3-dimensional side view from above of a manifold with an array of fluidic cells according to the embodiment of Fig. 4.
Fig. 2 shows a 3-dimensional side view of the system of the invention wherein the sensors (5) are contacting with a drop (10) generated in the fluidic cells. The enlargement shows the principle of ligand (52) capture of the analyte (53) at the tip of the sensors (5).
Fig. 3A and 3B shows section diagrams of a fluidic cell, wherein a stable drop (10) is generated for sample measurement.
Fig. 4A and 4B shows section diagrams of a fluidic cell, wherein a measurement well (40) is used for sample measurement.

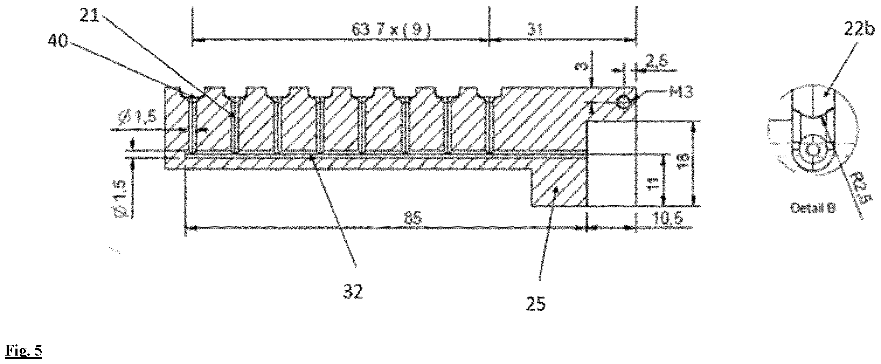

Fig. 5 shows construction diagrams of a manifold (20) according to of a particular embodiment of the system of the invention wherein a main distribution channel (32) in fluid connection with fluid distribution channels (21) is used.
Fig 6 shows a side view of a system of the invention wherein manifold (20) is implemented within an Octet RED96/Octet R system connected to an AmbR high-throughput automated bioreactor. The analysis module (65) is used to address fluid samples from a sample cup (64) to the manifold (20).
Fig. 7 shows the application of the invention to perform on-line monitoring of antibody titers in a vessel by bio-layer interferometry with the Octet RED96 system (60).
Fig. 8A shows the raw data obtained by the Octet RED96 system for all conducted analysis according to example 2.
Fig 8B shows the resulting outputs (antibody titers) evaluated over the 14h period by the Octet system and the theoretical antibody concentration in the vessel according to the concentration of the stock solution and the pump flow rate according to example 2.

References:
- 5: sensor
- 51: optical fiber
- 52: ligand
- 53: analyte
- 10: drop
- 11: Sample
- 20: manifold
- 21: fluid distribution channel
- 21a: outlet of the distribution channel
- 22a, 22b: collecting channel
- 25: body
- 26: main waste channel
- 30: fluid distribution tubing
- 31: fitting
- 32: main distribution channel
- 40: measurement well
- 50: automated bioreactor system61 vessel
- 62: liquid handling system
- 63: sampling device
- 64: sample cup
- 65: analysis module
- 66: syringe pump
- 67: stock vessel
- 68: pumps
- 69: medium
- 70: bio-layer Interferometry analyser - Octet R
- 71: well plate

### Example 1 - Manifold with stable sample drop

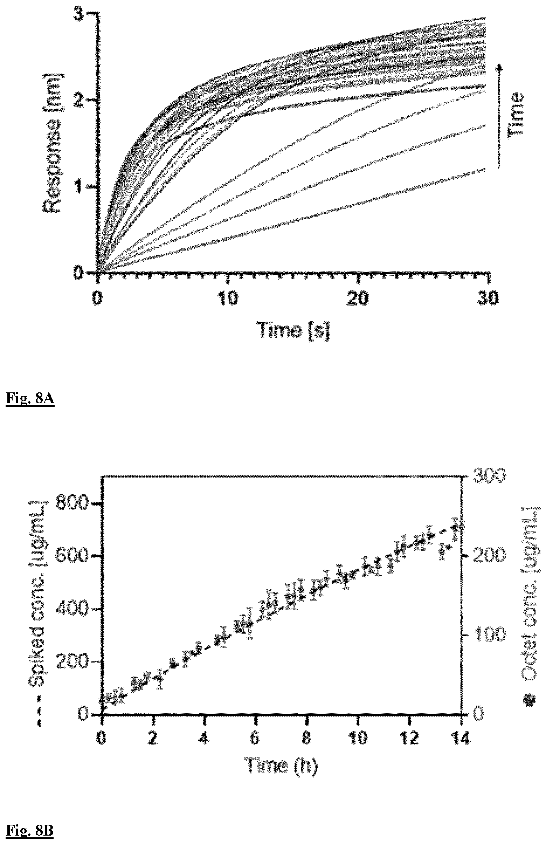

Fig. 8 shows an example of a production diagram for the body of a manifold with fluidic cells according to the embodiment of Fig. 3 with exemplary dimensions. In an example a manifold (20) comprising 8 fluidic cells was produced by machining PEEK. The manifold, the fluid distribution tubing (30) (PTFE Teflon) is fitted in each fluid distribution channel (21) so that it is emerging in the fluid collecting well (22).

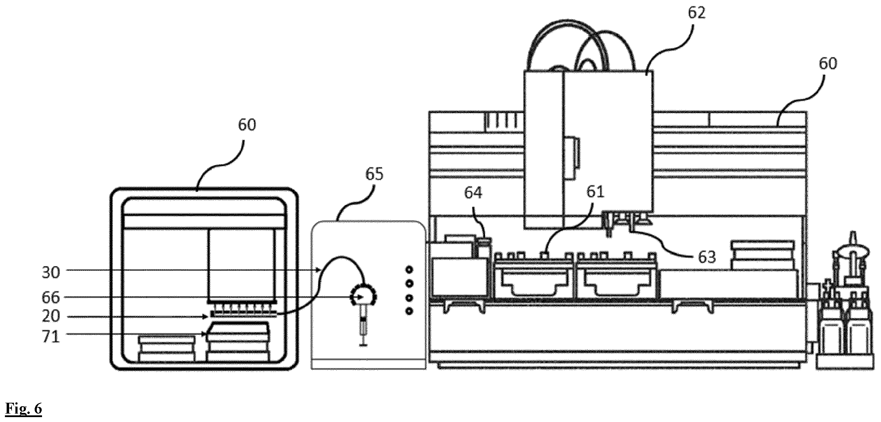

Fig. 6 shows the implementation of the manifold within an Octet RED96/Octet R system connected to an AmbR 15 high-throughput automated bioreactor system (60), equipped with a liquid handling system (62) that can automatically dispense and extracts sample fluid from the vessels (61), and can address them to the sample cup (64) of the analysis module (65). The analysis module (65) is used to automatically address fluid samples from a sample cup (64) to the manifold (20), using one syringe pump (66) for the provision of the sample. The manifold (20) was arranged in an Octet RED96/Octet R (60), comprising the sensors (5) and a well plate in a 96-wells format (71).

For the generation of the stable drop (10) the Octet RED96/Octet R was configured to position the sensors at a distance between the sensor and the outlet of the fluid distribution tubing (30) of approximatively 2 mms and the analysis module (61) was configured to push the adequate volume of fluidic sample into the fluid distribution tubing (30) over a predetermined distance.

### Example 2 - On-line monitoring of antibody titers

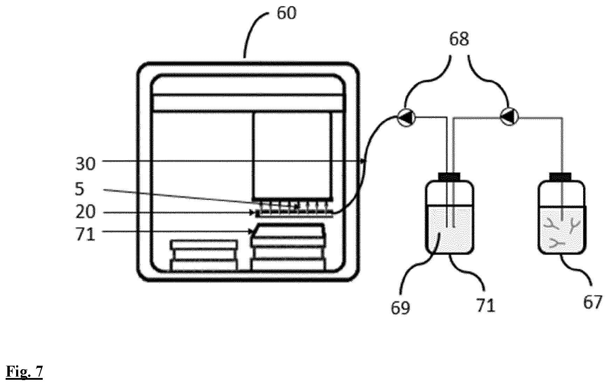

Fig. 7 shows the application of the invention to perform on-line monitoring of antibody titers in a vessel by bio-layer interferometry with the Octet RED96 system (70). Biosensors immobilized with protein A were used. Protein A can bind with strong affinity to the Fc portion of antibodies and is thus classically used as a ligand (52) for antibody quantitation. Regular increase of antibody titer in the vessel (61) was obtained by injecting antibody solution from a second stock vessel (67) of known concentration with the help of a pump (68) at a constant flow rate. The vessel initially contained only a defined volume of medium without antibodies. Additionally, the fluidic system comprised a second pump (68) in order to automatically and timely (every 15 mins) address fluid samples from the vessel to the manifold (20) placed in the Octet RED96 system, where the sensors (5) were automatically addressed for the given contact period in order to perform analysis.

Antibody quantitative assays were automatically performed in duplicate (2 sensors) every 15 mins, right after addressing fluid samples from the vessel (61), during a 14h time period. The contact period between protein A biosensors and the fluid samples was fixed to 30s. Calibration were performed in the well plate (71) every 1 h. Fig. 8A shows the raw data obtained by the Octet RED96 system for all conducted analysis. Fig 8B shows the resulting outputs (antibody titers) evaluated over the 14h period by the Octet system and the theoretical antibody concentration (dotted line) in the vessel according to the concentration of the stock solution and the pump flow rate.

## Claims

1. A system for measuring one or more analytes (53) in discrete fluid samples from a vessel adapted to contain a fluid comprising:
- one or more sensors (5) for the measurement of the analytes (53) in a contact volume of the fluid sample;
- an analysis module (55) comprising at least one main sampling line in fluidic connection with the vessel and one or more pumps in fluidic connection with said main sampling line(s) and adapted to selectively pump fluid from in the main sampling line away from the vessel;
- at least one fluid distribution tubing (30) in fluidic connection with the analysis module;
- a manifold (20) comprising a body (25), and in the body (25), for each sensor (5), one fluidic cell comprising one fluid distribution channel (21) and a collecting channel (22a, 22b) in fluidic communication with the fluid distribution channel (21);
wherein the fluid distribution tubing (30) is fitted in the fluid distribution channel (21) so that it is emerging in the collecting channel (22) and so that the sensor (5) can be contacted with at least a drop (10) of the fluid sample emerging at the top of the fluid distribution tubing (30) as the contact volume;
- or
wherein the fluid distribution tubing (30) is fitted in the fluid distribution channel (21) in fluidic connection with a measurement well (40), so the measurement well (40) can be filled with the fluid sample to create the contact volume and the sensor (5) can be dipped in the contact volume for measurement;
- and wherein said collecting channels (22a, 22b) are plunging so the fluid sample can be collected per gravity.

2. The system of claim 1, wherein the sensor is an optical sensor.

3. The system of claim 2, wherein the optical sensor is a biosensor.

4. The system according to one of the preceding claims, wherein a plurality of sensor is used.

5. The system according to one of the preceding claims, wherein the one or more fluid distribution channel (21) is in fluidic connection with a main distribution channel (32) in fluidic connection with the fluid distribution tubing (30).

6. The system according to one of the preceding claims, wherein the body (25) further comprises a main waste channel (26) or a main waste funnel (27) in fluidic connection with the collecting channels (22a, 22b).

7. The system according to one of the preceding claims, wherein said vessel is a fermenter, a bioreactor, a sampling cup, a sampling loop or any other vessel containing the sample fluid to be analyzed.

8. The system according to one of the preceding claims, further comprising an automated control system adapted to:
(a) configure said flow control system and store a sample filling configuration for the analysis module, the sample filling configuration comprising a prescribed period and / or a prescribed volume for creating the contact volume;
(b) automatically operate said sampling system in a sampling direction away from the vessel into the manifold (20) using the filling configuration;
(c) configure and store a contact configuration for the analysis module, in which sample flow is stopped for a prescribed contact period, during which an analysis is automatically launched and the sensor (5) is contacted with the contact volume;
(d) automatically operate said sampling system using the contact configuration; and / or
(e) configure and store a cleaning configuration for the system, preferred for the analysis module, the cleaning being achieved by pushing the sample fluid and / or cleaning fluid away from the sensor (5) into the collecting channels (22a, 22b), said cleaning configuration comprising one or more cleaning periods and / or one or more cleaning volumes;
(f) automatically operate the system using the cleaning configuration.

9. The system according to one of the preceding claims, further comprising a sensor positioning system, and wherein an automated control system is adapted to position the sensors (5) for contacting with the fluid sample by controlling the sensor positioning system.

10. A manifold (20) adapted to provide at least one contact volume of a fluid sample for contacting with at least one sensor (5) in a system for measuring analytes in said fluid sample, said manifold (20) comprising:
- a body (25),
- in the body (25), for each sensor (5), one fluidic cell comprising one fluid distribution channel (21) and a collecting channel (22a, 22b) in fluidic communication with the fluid distribution channel (21);
wherein a fluid distribution tubing (30) can be fitted in the fluid distribution channel (21) so that it is emerging in the collecting channel (22a, 22b) and so that the sensor (5) can be contacted with at least a drop (10) of the fluid sample emerging at the top of the fluid distribution tubing (30) as the contact volume;
or
wherein the fluid distribution tubing (30) can be fitted in the fluid distribution channel (21) in fluidic connection with a measurement well (40), so the measurement well (40) can be filled with the fluid sample to create the contact volume and the sensor (5) can be dipped in the fluid sample for measurement;
- and wherein said collecting channels (22a, 22b) are plunging so the fluid sample can be collected per gravity.

11. The manifold (20) according to claim 11, comprising a plurality of fluidic cells.

12. The manifold (20) according to one of the preceding claims, wherein the body (25) further comprises a main waste channel (26) or a main waste funnel in fluidic connection with the collecting channels (22a, 22b).

13. The manifold (20) according to one of the preceding claims, comprising a main distribution channel (32) in fluidic connection with the fluid distribution tubing (30) and with the one or more fluid distribution channel (21).

14. A method for measuring analytes in discrete fluid samples from a vessel adapted to contain a fluid comprising the steps of:
a) pumping the sample fluid from the vessel into a manifold (20) according to one of the claims 11 to 14 to create a contact volume of fluid sample in the fluidic cells of the manifold (20);
b) contacting the sensor (5) with the contact volume and conducting measurement for a contact period;
c) pushing the fluid sample into the collecting channels (22a, 22b) and eliminating the fluid sample per gravity;
d) outputting and / or forwarding measurement on a user interface or to an on-line process monitoring device.

15. The method of claim 15, further comprising a step e) cleaning the system by way of pumping sample fluid and / or cleaning fluid through the fluidic cell into the collecting channels (22a, 22b).

16. The method according to one of the preceding claims, wherein the method steps are reiterated in a timely defined manner so that in-process samples can be analyzed automatically

17. The method according to one of the preceding claims, wherein the process is a biological process.

18. The method according to one of the preceding claims, used for on-line process monitoring.

19. The method according to one of the preceding claims, used for process control, where the signal derived from sample analysis is used as an input variable in a process control model.
